# EUROPEAN PATENT SPECIFICATION

(11) **EP 3 557 261 B1**
(45) Date of publication and mention of the grant of the patent: **08.12.2021**
(21) Application number: 18168360.8
(22) Date of filing: 20.04.2018
(51) Int. Cl.: G01N 35/00

(54) **JUST IN TIME AVAILABILITY OF ANALYTICAL TEST RESULTS**
RECHTZEITIGE VERFÜGBARKEIT ANALYTISCHER TESTERGEBNISSE
DISPONIBILITÉ JUSTE À TEMPS DE RÉSULTATS DE TEST ANALYTIQUE

(43) Date of publication of application: 23.10.2019
(73) Proprietor: F. Hoffmann-La Roche AG, 4070 Basel (CH); Roche Diagnostics GmbH, 68305 Mannheim (DE)
(72) Inventor: DEPRAETERE, Louis, 1800 Vilvoorde (BE); LEFEVRE, Alexander, 1800 Vilvoorde (BE); PAERSSINEN, Janne-Henrik, 1800 Vilvoorde (BE); TOURNY, Annick, 1800 Vilvoorde (BE); VANDERKEEL, Marc, 1800 Vilvoorde (BE); VANDEWEYER, Katleen, 1800 Vilvoorde (BE); VERCAMMEN, Werner, 1800 Vilvoorde (BE)
(74) Representative: Ko, Benjamin Hyensuk

(56) References cited:
- EP-A1- 3 182 133
- WO-A1-2005/101023
- WO-A1-2017/114713

## Description

### FIELD OF THE INVENTION

The application relates to a computer implemented method for operating an analytical laboratory for processing biological samples. The application further relates to an analytical laboratory for processing biological samples as well as a computer program product comprising instructions which, when executed by a computer system, control an analytical laboratory to perform said method.

### BACKGROUND OF THE INVENTION

In vitro analytical diagnostic testing has a major effect on clinical decisions, providing physicians with pivotal information. Statistics reveal that a very high proportion of all physician decisions are dependent on in vitro diagnostic testing.

In analytical laboratories, in particular clinical laboratories, a multitude of analyses on samples are executed by an analytical system in order to determine the physiological state of a patient. The kind of analytical test to be executed on a biological sample is typically specified as a test order which is typically registered in a laboratory information system as a record and sent to the analytical laboratory. According to established laboratory procedures, when a biological sample is received, it is first identified - for example, by an identifier label and corresponding label reader such as a barcode label and barcode reader. Once the biological sample is identified, an order list is retrieved from a database comprising a plurality of test orders, each test order defining one or more processing steps to be carried out on the biological sample by one or more of the laboratory instruments. These processing steps may be pre-analytical processing steps (such as aliquoting, sample preparation), analytical processing steps (such as an assay to determine the presence and/or concentration of an analyte in the biological sample) or post-analytical processing steps (such as archiving of the biological sample). Before the biological sample can be processed by the various laboratory instruments of the analytical laboratory, a processing workflow is determined. The processing workflow is defined as a sequence in which the test orders are to be processed and/or a timing of processing of the test orders.

According to current practice, samples, respectively test orders which need to be processed and analyzed very urgently - as the analysis result may be of life-critical importance for a patient - are marked as urgent STAT samples. STAT laboratory tests and services are those that are needed immediately in order to manage medical emergencies. According to current laboratory practice, STAT test requests are given the highest priority by the analytical laboratory for processing, analysis and reporting.

Health care institutions (such as hospitals, clinics, medical practices, home-based offices of medical practitioners etc.) have to manage a number of limited medical practitioners (e.g. physicians, nurses, etc.) to be able to treat an ever increasing number of patients efficiently and effectively. Uncertain time of availability of results of analytical test leads to lack of efficiency as physicians have to wait on results of analytical test(s) to become available to be able to determine a diagnosis/ treatment or need for further analytical tests, respectively medical practitioners need to check multiple times whether results for an analytical test have become available for the most urgent patients, or go through a long list of already processed lab results and related alerts - that they might not have had time to review yet.

Furthermore, not only uncertain time of availability of results of analytical test(s), but in addition, late/ delayed availability of results of analytical tests are a cause of lack of efficiency of such health care institutions. This uncertainty regarding time of arrival of results in an increased risk of a negative outcome for the patient. In order to ensure timely availability of results of analytical test(s), according to currently known practice, physicians would order analytical test(s) on a sample of a patient as urgent STAT whenever they decide (based on certain rules or personal judgement) that the particular analytical test or patient sample should be prioritized. Factors influencing the physician's decision to classify a sample or test order (for analytical test) as urgent include - but not limited to - patient severity (or indication thereof) and/or patient history. However, if less urgent tests are also ordered STAT or if too many urgent test orders are issued in a timeframe, a backlog of the analytical laboratory may develop if the number of urgent test orders exceeds the throughput of the laboratory and hence each sample will be delayed, including urgent ones.

WO2017/114713 relates to processing and delivering cells to a therapy patient. It proposes a system that is inter alia configured to determine if variations from a processing protocol have occurred that alter a sample processing timeline based at least in part on a time of receipt of an identification signal, and to provide one or more updated estimated completion times of the processing protocol. The sample processing timeline may be an estimate or forecast model of a completion time for the sample based on empirical or historical estimates for a particular production plan. The forecast may also take into account estimated resource availability for the scheduled sample based on concurrent production of other samples in the facility. In the context of sample processing, an example is mentioned in which, if an operator is not available but an incubator is available, the sample has to wait until both resources are available. In one embodiment, the availability of the resources are affected by the number of resources assigned to the overall process, their working conditions such as working hours and/or unavailability due to breakdowns or repair.

There is a need for an analytical laboratory, respectively a method of operating an analytical laboratory in such a way as to allow for efficient use of resources of health care institutions to improve patient care and/or reduce costs.

### SUMMARY

It has been observed that there are situations in patient care institutions, when a medical practitioner (and patient) has to wait for result(s) of analytical test order(s) longer than expected, while at the same time results of other analytical test orders are available well before a medical practitioner actually has time to consider them.

Results of analytical tests which are not available when a medical practitioner would need them (in order to diagnose and/or treat a patient, to decide on further analytical testing, etc.) shall be hereinafter referred to as delayed results.

Results of analytical tests which are available before they are needed/ considered by a medical practitioner shall be hereinafter referred to as early results.

The main reason for a test order to lead to an early result is that medical practitioners of a health care institution order analytical tests as urgent if they judge (based on certain rules or personal judgement) that the particular analytical test or patient sample should be prioritized, but lack resources (most commonly the time of medical practitioners) to consider these when the results are available. It has been observed that multiple times more test orders are ordered as urgent compared to the number of actual emergency cases in a typical health care institution. In other words, early results are just sitting idle until the medical practitioner has time to consider them. Also, medical practitioners may order a laboratory test around the end of their work shift and hence often the results are available early as the medical practitioners are not working anymore by the time the test results become available.

Inventors of the present disclosure recognized that early results hold back the processing of other more urgent test orders. In other words, inventors of the present disclosure recognized that occurrence of delayed results can be greatly reduced by the analytical laboratory re-evaluating the priority level (urgent STAT vs. normal; or less urgent than STAT) of the analytical test orders/ samples as defined at ordering (e.g. by the medical practitioner). For such re-evaluation of priority level by the analytical laboratory, a forecast of expected time of availability of test results is calculated for each analytical test order followed by an adjustment of the processing priority thereof based on the staff workload/ backlog at the time of forecast availability. The term staff workload/ backlog refers to the sum of all actions, resources needed before the analytical results are actually considered. The staff workload/ backlog may represent the number of other patients a medical practitioner must handle before having time to treat/diagnose the patient in question. The staff workload/ backlog may also represent the period of time until a medical practitioner starts his/her work shift.

Potentially life-critical test results are reviewed by the medical practitioner/ or proxy immediately - hence the staff workload/ backlog vs. the current test order is always zero/ empty. In other words, treatment of patients in life-critical situations - and thus also consideration of related analytical test orders - are always handled first.

At onset - when the analytical laboratory retrieves a test order, the priority level of an analytical test corresponds to the priority level set by the orderer, mostly a medical practitioner. The processing priority of an analytical test order is reduced if at the time of forecast expected availability of the test results the medical practitioner will already have a high staff workload/ backlog. On the other hand, if the staff workload/backlog of the medical practitioner at the forecast expected availability of the test results is low (meaning the medical practitioner is most likely to need the results right away, otherwise valuable time is wasted), the processing priority of an analytical test order is raised or maintained if already set to urgent. According to particular embodiments disclosed herein, the forecast of expected time of availability of test results is recalculated for each analytical test order and the priority level of all test orders are re-assessed in an iterative manner each time the processing priority of a test order is changed. In another words, the impact of each change of processing priority of a test order on the forecast of all analytical tests is assessed.

As often laboratory personnel needs to process (e.g. validate) the test results before being available to the medical practitioner, to account for possible delays due to such processing by laboratory personnel, according to embodiments disclosed herein, in addition to the staff workload/ backlog of medical practitioner, the staff workload/ backlog of laboratory personnel is taken into account in scheduling of the processing workflow of analytical test orders as well as in calculating the forecast availability of the test results.

All in all, the analytical laboratory, respectively method of operating an analytical laboratory disclosed herein enable significantly more efficient operation of health care institutions by accurate forecasting of availability of laboratory test result(s) in combination with staff workload / backlog based prioritization and scheduling of the processing workflow of analytical test orders.

Embodiments of the disclosed method/ system are particularly advantageous since the occurrence of delayed results can be significantly reduced without any additional investments such as increasing throughput of the analytical laboratory (e.g. by additional instruments, higher throughput instruments, etc.). In other words, a traditional first come first served approach with some pre-defined prioritization of processing test orders for analytical tests is substituted with a prioritization corresponding to the actual needs of the health care institution. Reduction of delayed results as well as increasing the forecasting accuracy of test result availability lead to a significantly better quality of patient care (reduced length of stay of a patient in a health care institution due to timely and/or quicker delivery of life-critical test results - in particular in emergency care as well as financial benefits (better use of existing resources, reduced cost due to less time spent in a health care establishment by patients). In addition, embodiments disclosed herein support health care institutions to better cope with peaks of patient influx (epidemics, bio-terrorism) in that timely availability of analytical results ensures healthy patients can be released sooner, thereby freeing up resources. Due to the uncertain time of availability of results of analytical test, medical practitioners often perform point of care analytical testing instead of issuing a test order to an analytical laboratory. However, point of care analytical testing costs significantly more than the same analytical test performed by an analytical laboratory. Furthermore, point of care testing sometimes provides less accurate analytical results as compared to laboratory testing. By reducing the uncertainty of availability of analytical test results, embodiments herein disclosed further contribute to the reduction of operational costs and better quality of patient care since medical practitioners are less motivated to replace laboratory testing with point of care testing.

In addition, reporting a forecast of expected time of availability of test results supports medical practitioners of health care institutions to much better plan their activities. Better planning of activities of medical practitioners inherently increases cost effectiveness and also improves the quality of care as timely availability of results of analytical tests has a significant impact on decisions influencing patient care. Timely availability of results of analytical tests allows for speedier treatment and may reduce the risks of complications, hence improving patient care. On the other hand, timely availability of results of analytical tests also contributes to reducing costs as patients with less severe conditions may leave the health care institution earlier, while patients with more severe conditions are diagnosed earlier which very often is critical in effectively treating severe conditions.

According to further embodiments disclosed herein, the processing priority level of a test order is adjusted also based on a processing status of the test order to account for unexpected events, such as low sample quality, rejected test result in need for a rerun of the analytical test, hardware failures of an instrument, etc.

According to further embodiments disclosed herein, the processing priority level of a test order is adjusted also based on the type of test order to ensure that analytical tests which are known to be required for life-critical diagnoses are performed with highest priority, e.g. analytical tests needed to diagnose a heart-attack, an imminent sepsis, etc.

According to embodiments disclosed herein, the forecasting of availability of laboratory test results is based on parameters which are revised based on feedback indicative of actual time of availability of results of past test orders.

### BRIEF DESCRIPTION OF THE DRAWINGS

Further characteristics and advantages of the disclosed method / device /system will in the following be described in detail by means of the description and by making reference to the drawings which show:
- Fig. 1: a flowchart illustrating an embodiment of the disclosed method for operating an analytical laboratory;
- Fig. 2: a flowchart illustrating an embodiment of the disclosed method for operating an analytical laboratory wherein the processing priority of a test order is set initially based on an objectively determined urgency level;
- Fig. 3: a flowchart illustrating an embodiment of the disclosed method for operating an analytical laboratory wherein machine learning is employed to revise parameters for determining forecast availability of analytical results if the actual availability of analytical result(s) deviate from the forecast availability;
- Fig. 4A, 4B: a flowchart illustrating an embodiment of the disclosed method for operating an analytical laboratory, wherein the biological sample(s) is transferred to a further analytical laboratory if the forecast availability of analytical result(s) corresponding to the test order(s) for that biological sample is beyond a specified availability deadline;
- Fig. 5: a flowchart illustrating an embodiment of the disclosed method for operating an analytical laboratory, wherein a phlebotomist is alerted to collect a new patient sample if at least one analytical step of the test order(s) cannot be performed on a biological sample due to sample quality/ quantity;
- Fig. 6: a flowchart illustrating an embodiment of the disclosed method for operating an analytical laboratory, wherein the forecast availability(s) of analytical result(s) is reported when being determined and redetermined;
- Fig. 7: a flowchart illustrating an embodiment of the disclosed method for operating an analytical laboratory, wherein the processing priority level of all test orders is set to the highest level amongst the test orders corresponding to the same patient, same doctor and/or same hospital;
- Fig. 8: a highly schematic block diagram of an embodiment of the disclosed analytical laboratory;
- Fig. 9: a mockup of an example of a mobile device application reporting the forecast availability of analytical results in the form of progress bars.

### DETAILED DESCRIPTION

Certain terms will be used in this patent application, the formulation of which should not be interpreted to be limited by the specific term chosen, but as to relate to the general concept behind the specific term.

The term **'laboratory instrument'** as used herein encompasses any apparatus or apparatus component operable to execute one or more processing steps / workflow steps on one or more biological samples. The expression 'processing steps' thereby refers to physically executed processing steps such as centrifugation, aliquotation, sample analysis and the like. The term 'laboratory instrument' covers pre-analytical instruments, post-analytical instruments and also analytical instruments.

The term **'pre-analytical instrument'** as used herein comprises one or more lab-devices for executing one or more pre-analytical processing steps on one or more biological samples, thereby preparing the samples for one or more succeeding analytical tests. A pre-analytical processing step can be, for example, a centrifugation step, a capping-, decapping- or recapping step, an aliquotation step, a step of adding buffers to a sample and the like. The expression **'analytical system'** as used herein encompasses any monolithic or multi-modular laboratory device comprising one or more lab-devices or operative units which are operable to execute an analytical test on one or more biological samples.

The term **'post-analytical instrument'** as used herein encompasses any laboratory instrument being operable to automatically process and/or store one or more biological samples. Post-analytical processing steps may comprise a recapping step, a step for unloading a sample from an analytical system or a step for transporting said sample to a storage unit or to a unit for collecting biological waste.

The term **'analyzer'** / **'analytical instrument'** as used herein encompass any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, tissue analyzers (incl. morphological stainers and histochemical stainers) used to detect the result of chemical or biological reactions or to monitor the progress of chemical or biological reactions.

The term **'analytical laboratory'** as used herein encompasses any system for the use in a laboratory comprising a plurality of laboratory instruments operatively connected to a control unit.

The term **'control unit'** as used herein encompasses any physical or virtual processing device configurable to control an analytical laboratory comprising a plurality of laboratory instruments in a way that a workflow and workflow step(s) are conducted by the analytical laboratory. The control unit may, for example, instruct the analytical laboratory (or a specific instrument thereof) to conduct pre-analytical, post analytical and analytical workflow(s)/ workflow step(s). The control unit may receive information from a data management unit regarding which steps need to be performed with a certain sample. In some embodiments, the control unit might be integral with a data management unit, may be comprised by a server computer and/or be part of one instrument or even distributed across multiple instruments of the analytical laboratory. The control unit may, for instance, be embodied as a programmable logic controller running a computer-readable program provided with instructions to perform operations.

The term **'communication network'** as used herein encompasses any type of wireless network, such as a WIFI, GSM, UMTS or a cable based network, such as Ethernet or the like. In particular, the communication network can implement the Internet protocol IP. For example, the communication network comprises a combination of cable-based and wireless networks. In embodiments wherein units of the system are comprised within one laboratory instrument, the communication network comprises communication channels within an instrument.

The term '**user interface'** as used herein encompasses any suitable piece of software and/or hardware for interactions between an operator and a machine, including but not limited to a graphical user interface for receiving as input a command from an operator and also to provide feedback and convey information thereto. Also, a system /device may expose several user interfaces to serve different kinds of users/ operators.

The term **'workflow'/** '**processing workflow'** as used herein refers to a collection of workflow steps /processing steps. According to particular embodiments, the workflow defines a sequence in which the processing steps are carried out.

The term '**workflow step'** or '**processing step'** as used herein encompasses any activity belonging to a workflow. The activity can be of an elementary or complex nature and is typically performed at or by one or more instrument(s).

The terms **'sample', patient sample'** and '**biological sample'** refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

A **'STAT sample'** is a sample which needs to be processed and analyzed very urgently as the analysis result may be of life-critical importance for a patient. STAT laboratory tests and services are those that are needed immediately in order to manage medical emergencies.

The term **'sample tube'** refers to any individual container for transporting, storing and/or processing a sample. In particular, said term without limitation refers to a piece of laboratory glass- or plastic-ware optionally comprising a cap on its upper end.

The term '**analyte**' as used herein refers to a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Thus 'analyte' is a general term for substances for which information about presence and/or concentration is intended. Examples of analytes are e.g. glucose, coagulation parameters, endogenic proteins (e.g. proteins released from the heart muscle), metabolites, nucleic acids and so on.

The term **'analysis'** or **'analytical test'** as used herein encompasses a laboratory procedure characterizing a parameter of a biological sample, e.g. light absorption, fluorescence, electrical potential or other physical or chemical characteristics of the reaction to provide the measurement data.

The term **'test order'** as used herein refers to any data object, computer loadable data structure, modulated data representing such data being indicative of one or more laboratory processing steps to be executed on a particular biological sample. For example, a test order record may be a file or an entry in a database. According to embodiments disclosed herein, a test order can indicate a test order for an analytical test if, for example, the test order comprises or is stored in association with an identifier of an analytical test to be executed on a particular sample. Alternatively or additionally, the test order may refer to pre- and/or post-analytical processing steps to be performed on the biological sample.

The terms **'analytical result',** '**test result'** or '**result of analytical test'** as used herein encompasses any data that is descriptive of a result of a measurement of an analyte in a biological sample, namely qualitative and/or quantitative measurement of an analyte in a sample. In particular, the analytical data comprises an identifier of the sample for which the analysis has been performed and data being descriptive of a result of the analysis, such as measurement data. Furthermore, the terms **'analytical result',** '**test result'** or '**result of analytical test'** also encompass calculated results based on measurement of an analyte in a biological sample, as risk scores or other values calculated using various clinical decision support algorithms.

Particular embodiments of the disclosed method/ system shall be described as follows with reference to the figures.

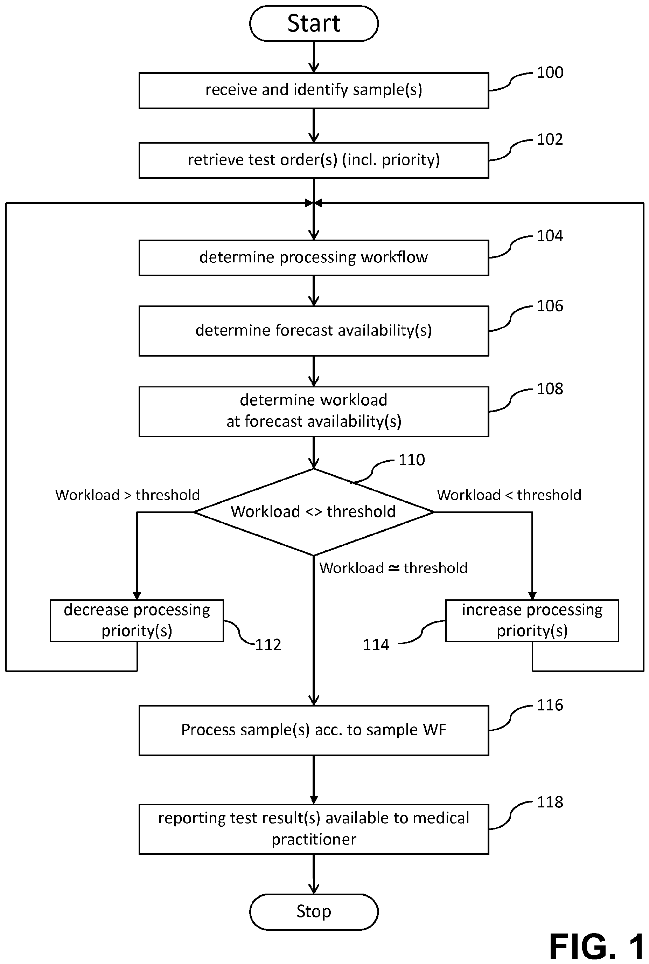

A first embodiment of the method for operating an analytical laboratory 1 shall be now described as illustrated on the flowchart of figure 1.

In a **step 100,** one of the plurality of laboratory instruments 10 of the analytical laboratory 1 receives and identifies the biological sample. The biological sample is identified, in particular based on an identification label attached to a sample tube holding the biological sample. The identification label attached may be a barcode, an RFID tag, or the like.

Once the biological sample is identified, in **step 102** the control unit 20 retrieves one or more test orders corresponding to each biological sample, each test order defining at least one analytical step to be carried out on the biological sample by at least one of the plurality of the laboratory instruments 10 . According to particular embodiments of the disclosed method/ system, several processing steps may be carried out at the same test order, such as several aliquots being prepared from the biological sample; or several analytical tests being carried out by the same analytical instrument 10.

According to embodiments disclosed herein, the test orders are retrieved by the control unit 20 from an internal storage (such as a database), from a laboratory information system LIS and/or from a user interface communicatively connected to the control unit 20 or from a hospital information system HIS 50.

Each test order comprises a processing priority indicative of a precedence by which the test order is to be carried out with respect to other test orders. As one example, processing priority may be defined on a two value scale such as "Urgent" and "Normal", wherein increasing a priority from "Normal" results in "Urgent" and decreasing a priority from "Urgent" results in "Normal", "Urgent" being considered in this case the highest level or processing priority. Alternatively, or additionally, the processing priority may be defined as a numerical scale, e.g. from 1 to 10, 10 being the highest priority level and 1 the lowest.

According to embodiments disclosed herein, when tests are ordered (usually by a medical practitioner), the processing priority is set either as 'normal' or 'urgent/STAT', STAT orders for analytical tests being issued for those tests that are needed immediately in order to manage medical emergencies.

In **step 104,** the control unit 20 determines a processing workflow for processing the biological samples based on corresponding test order(s) and corresponding processing priority(s). The processing workflow defines a list, in particular a sequence of processing steps to be carried out by the plurality of laboratory instruments 10 in order to complete the test orders.

According to embodiments of the disclosed method/system, the processing workflow also defines one or more of the following:
- Sequence in which the test orders are to be processed.
   The test order processing sequence defines the order in which the biological samples are processed by the test orders.
- Timing of processing of the test orders
   According to embodiments of the disclosed method/ system, in addition to the processing sequence, the timing according to which the biological samples or aliquots thereof are processed is also defined by the processing workflow .

All-in-all, the processing workflow for processing each biological sample is determined such that test order(s) with higher processing priority are scheduled to be completed before test order(s) with lower processing priority. It must be emphasized that due to unforeseeable events, such as an instrument malfunction, the actual processing might deviate from the processing workflow in terms of time of availability of the results and/or in the sequence of the processing steps.

In a **step 106,** the availability of analytical result(s) corresponding to each test order is determined. The availability of analytical result(s) in this context refers to the point in time when the control unit 20 predicts/ forecasts that the at least one analytical step on the biological sample is completed and the corresponding result becomes available. According to various analytical tests, an analytical result is not immediately available after completion of the analytical step(s) on the biological sample, but only after a so-called validation of the measurement. In such cases, the forecast availability of analytical result(s) takes into account the time required for such validation.

According to embodiments disclosed herein, the step 106 of determining a forecast availability of an analytical result comprises one or more substeps from the list comprising:
- Determining a predicted instrument processing time
   This substep refers to predicting the total time required by the laboratory instrument(s) 10 to process the biological sample to complete the corresponding test order. According to embodiments disclosed herein, the algorithm predicting the instrument processing time takes into account expected staff workload, throughput of instruments, downtimes due to scheduled and/or predicted maintenance activities. In particular, historical data indicative of past processing time is taken in consideration for predicting instrument processing time.
- Determining a predicted validation time
   The validation time is determined in view of a schedule of a laboratory staff responsible for validation of analytical test results. In other words, the validation time is correlated with the work schedule of the staff that performs the validation - at the time when the processing of the biological sample by the laboratory instrument(s) 10 is completed. In the current context, the term 'schedule of a laboratory staff' encompasses data representing work shifts of laboratory staff, periods of increased workload/ demand. According to embodiments disclosed herein, the algorithm predicting the validation time takes into account time of day, day of week, holidays, periods with increased occurrence of epidemics, etc. A degree of automation of result validation is taken in consideration for predicting validation time.
- Determining a predicted sample transportation time
   This substep comprises prediction of the period of time required for the biological sample(s) to be transported from a point of collection from the patient (e.g. by a phlebotomist) to the analytical laboratory 1 . According to embodiments disclosed herein, the algorithm predicting the sample transportation time takes into account time of day, day of week, holidays, periods with increased occurrence of epidemics with higher than usual demand, traffic rush hours, etc.
- Determining delays due interdependency(s) between analytical step(s) and analytical results
   Certain analytical steps are performed based on/ in reaction to results from other analytical steps. For example, reflex testing, reruns, confirmatory testing. These interdependencies are taken in consideration in predicting forecast availability of an analytical result.

In a **step 108,** staff workload is determined at the time of the forecast availability(s) of the respective analytical test result(s). According to various embodiments, staff comprises a physician responsible for diagnosing/ treating the patient whose biological sample the respective analytical test result(s) correspond to. Within step 108, three substeps can be identified:
- I) Identifying the relevant staff for a particular analytical result
   According to embodiments disclosed herein, the relevant staff for a particular analytical result is retrieved by the control unit 20 from a Hospital Information System HIS 50 and/or part of a test order and/or determined by a schedule of the health care institution. For example, in an emergency ward, the responsible staff comprises all physicians on duty at the time of the forecast availability(s). On the other hand, for a specialist physician's office, the responsible staff comprises all physicians of a respective specialty or even a particular physician treating/ diagnosing the respective patient. In very simple terms, in this substep the control unit 20 determines who is "waiting" for this result.
- II) Predicting the staff workload of the relevant staff
   In this substep, a staff workload of the relevant staff is determined for the moment in future when the respective analytical result(s) is forecast to become available. The algorithm determining the staff workload looks ahead instead of merely relying on a current staff workload. This ensures that the prediction of the staff workload is still relevant when the samples have been processed, the measurements validated and the results available for the staff.
   This determination is used to evaluate the actual processing priority of a test order. For example, if a medical practitioner will still have a high number of patients to consult before the patient corresponding to a particular test order, then the respective analytical results can "wait". On the other hand, if by the time an analytical result is forecast to be available the medical practitioner would have no other patients to be treated/ diagnosed, then the test order would be prioritized as otherwise the medical practitioner would have to wait for the test results which would be a waste of time and resources and potentially a risk for the patient.
- III) Determining a workload threshold
   The workload threshold is indicative of a staff workload limit under which the test order is to be prioritized to avoid a test result from being delayed and above which the test order is to be de-prioritized to allow for more urgent test orders to be completed first. A workload threshold above 0 acts as a sort of buffer to allow for some imprecision of the forecasting of result availability and still avoiding delayed respectively too early test results. A workload threshold of 0 leads to just in time availability, but with no tolerance of the predictions.
   Another way to describe the workload threshold is to say that the medical practitioner should have the analytical results available a certain amount of time (a certain number of patients still to be treated) before he becomes free.

According to embodiments disclosed herein, the staff workload and the workload threshold are indicative of one or more from the list comprising:
- A number of "other" test results a medical practitioner is to review before reviewing the test result corresponding to the respective test order
   In other words, the future backlog of medical practitioner is determined. The term "other" in this context refers to any test result different from the one for which the staff workload is currently being determined for.
- A predicted period of time from the forecast availability of analytical result(s) until a medical practitioner is to review the analytical result(s)
   The period of time before a medical practitioner is predicted to review the analytical result(s) is determined based on a number of factors such as, but not limited to: a work schedule of the medical practitioner, work shifts, foreseen absences and/or more urgent planned activities.
   Since patients in critical condition are always diagnosed and/or treated first, the staff workload corresponding to a test order for the biological sample of a patient in critical condition is always 0 or 'empty', wherein a staff workload of 0 ensures that the processing priority of corresponding test orders are increased to the highest level of processing priority.
- The severity of the patients of the medical practitioner
   In addition to the number of the test results a medical practitioner is to review, the outcome of these respective test results is also taken in consideration in calculating the staff workload of a medical practitioner. For example, if a medical practitioner has a high number of "bad" test results indicating very serious patient medical conditions, then his/her staff workload is considered to be higher versus a doctor with his/her patient(s) showing "good" test results (i.e. no serious medical conditions). Since treating a serious condition takes more time. The distinction between severe and less severe conditions is determined according to know ranges for analytical tests results which aid to objectively determine whether the doctor has a serious case to solve (that takes more time).

According to further embodiments, the step of determining the staff workload comprises the step of determining a "workload overflow" from a medical practitioner ending a work shift to another medical practitioner starting his/her work shift. The workload overflow is added to the staff workload of the medical practitioner starting the work shift in that the test order is assigned (even if temporarily) to more than one medical practitioner. Hence the workload is not empty necessarily empty at the start of a work shift.

In a next **step 110,** the control unit 20 compares the staff workload and the workload threshold:
- If - at the time of forecast availability of the analytical result(s) - the staff workload is higher than the workload threshold, then the processing priority corresponding to the respective test order is decreased in **step 112.** This case corresponds to a medical practitioner (doctor) with a backlog who will already have patients to diagnose/treat before needing this analytical result. Hence the test order is de-prioritized to allow for more urgent test orders to be processed by the analytical laboratory. It shall be noted that a processing priority which is already at the lowest level in the defined range is in this case maintained at this lowest level.
- On the other hand, if - at the time of forecast availability of the analytical result(s) - the staff workload is lower than the workload threshold, then the processing priority corresponding to the respective test order is increased in **step 114.** This case corresponds to a use case when the medical practitioner is predicted to need the analytical results immediately or very shortly after they are forecast to become available. For example, if the staff workload threshold is defined as two patients and at the time of forecast availability, the medical practitioner only has one other patient waiting to be treated/ diagnosed, that means that the analytical result will be needed in very short time after becoming available. Actually in less time than the defined threshold (buffer). Therefore, the processing is increased.
- Once the staff workload matches the staff workload threshold, in **step 116** the control unit 20 controls the plurality of laboratory instruments 10 of the analytical laboratory 1 to process each biological sample according to the processing workflow. The term "match" is to be interpreted, in the context of a staff workload matching the staff workload threshold, as being substantially equal to.
   Example 1: for a medical practitioner treating an average of 4 patients per hour, an average forecasting deviation of 30 mins, then a threshold of 2 patients would be determined. Hence if the workload of the medical practitioner is 5 patients, it means the results would be sitting idle anyhow, therefore the test order may be deprioritized. On the other hand, if the workload of the medical practitioner is 1 patient, it means the results have a risk to be available too late, therefore the test order should be prioritized. Example 2: When a medical practitioner starts his/her work shift at 8AM, the workload is 0 or "empty" and the forecast availability of the analytical results is 8:30AM, this means the test order should be prioritized. However, if the the forecast availability of the analytical results is 1:30AM, then the analytical result would be early as the medical practitioner would be out of office and the test order can be deprioritized.
   Note: the above examples assume patients in non-critical condition.
- The test results obtained by the at least one analytical step on the biological samples performed by the at least one of the plurality of the laboratory instruments 10 are then reported **step 118.** According to various embodiments disclosed herein the results are reported by means of a data transfer to a hospital information system HIS 50, via a user interface of the control unit 20, by a data transfer to a cloud server accessible by a mobile device application of a medical practitioner, by way of a printout or other suitable means.

According to further embodiments disclosed herein, the step 116 of controlling the plurality of laboratory instruments 10 of the analytical laboratory 1 to process each biological sample according to the corresponding processing workflow comprises the step of controlling an instrument for loading the biological samples such as to load the biological samples in the order of processing priority(s) thereof, in order to avoid samples with lower processing priority to overload the laboratory instruments 10 and samples with higher priority being stuck.

In an embodiment of the analytical laboratory 1 wherein sample tubes comprising the biological samples are loaded into an instrument for loading samples in bulk, the instrument for loading samples is configured to sort the sample tubes based on the processing priority of the corresponding test orders.

In a further embodiment of the analytical laboratory 1 wherein sample tubes comprising the biological samples are loaded into an instrument for loading samples in sample racks, the instrument for loading samples is configured to individually load (pick) the sample tubes from the sample racks based on the processing priority of the corresponding test orders.

As illustrated on the flowcharts of figures 1 through 7, according to embodiments disclosed herein, the processing workflow for processing each biological sample as well as the forecast availability of an analytical result corresponding to each test order are re-determined each time the processing priority of one or more test order(s) is changed, i.e. increased or decreased. In other words, the method is performed in iterations until an optimal solution is determined. In this context, iteration refers to the series of steps: 104 through 110. According to embodiments disclosed herein, a maximum number of iterations is defined after which the plurality of laboratory instruments 10 of the analytical laboratory 1 are controlled by the control unit 20 to process each biological sample according to the corresponding processing workflow even if the staff workload does not match precisely the staff workload threshold.

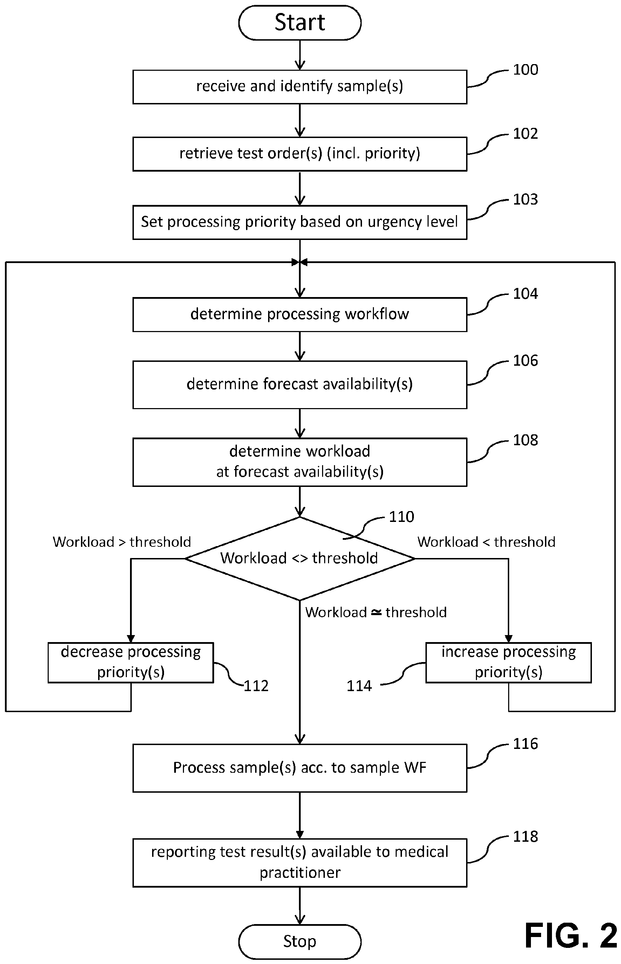

Figure 2 shows a flowchart illustrating an embodiment of the disclosed method wherein the processing priority of a test order is set - in **step 103** - based on a determined urgency level. The urgency level is determined by the control unit 20 based on one or more from the list comprising:
- Processing priority level of test order as retrieved
   In order to ensure timely availability of results of analytical test(s), medical practitioners would order analytical test(s) on a sample of a patient as urgent whenever they decide that the particular analytical test or patient sample should be prioritized. This choice of priority by the medical practitioner is weighted in when determining the actual urgency level.
- An indication of patient criticality
   Test orders related to patients in critical condition (e.g. heart attack, sepsis, stroke) are always prioritized and the urgency level is determined accordingly. The indication of patient criticality may be either part of the test order or retrieved separately by the control unit 20, in particular from a Hospital Information System HIS 50. Patient demographics is also a factor influencing test order urgency.
- Analytical test class
   Certain types or classes of analytical tests are known to be ordered in life-threatening conditions. Therefore, the control unit 20 determines the urgency level and sets the processing priority accordingly - irrespective of staff workload. In addition, certain dependencies between various analytical tests can also influence test order urgency.
- Patient condition progress/change
   If the condition of a patient deteriorates, the control unit 20 re-determines the urgency level and sets the processing priority at its highest accordingly. Patient condition progress/change may be determined in particular using active patient monitoring devices communicatively connected to a Hospital Information System HIS 50, from where it can be retrieved by the control unit 20. For example, active patient monitoring comprises (but not limited to): Connected ECG devices, respiratory function monitoring devices, blood oxygen level monitoring, pulse oximetry, arterial blood pressure monitoring, central venous pressure monitoring, continuous central venous oxygenation saturation monitoring, temperature monitoring. The patient monitoring can be either in a hospital setting or outside (including but not limited to patient home monitoring and patient monitoring in an ambulance). An advantage of considering patient condition using active patient monitoring is that once the patient's condition significantly deteriorates, test orders for that patient are automatically prioritized without the treating doctor having to contact the analytical laboratory.
- Source of test order
   There are certain use cases when the origin/ source of a test order plays an important role in determining the urgency and therefore necessary processing priority of a test orders.
- A status of one or more of the plurality of laboratory instruments 10 Changes in the operational status of laboratory instruments 10 directly affect the availability of analytical results. Hence these are considered in setting the urgency and hence processing priority of test order. For example, if one of two instruments 10 of the same kind malfunctions, then some test orders are de-prioritized to ensure the most urgent test are completed in time - despite the malfunctioning instrument 10.

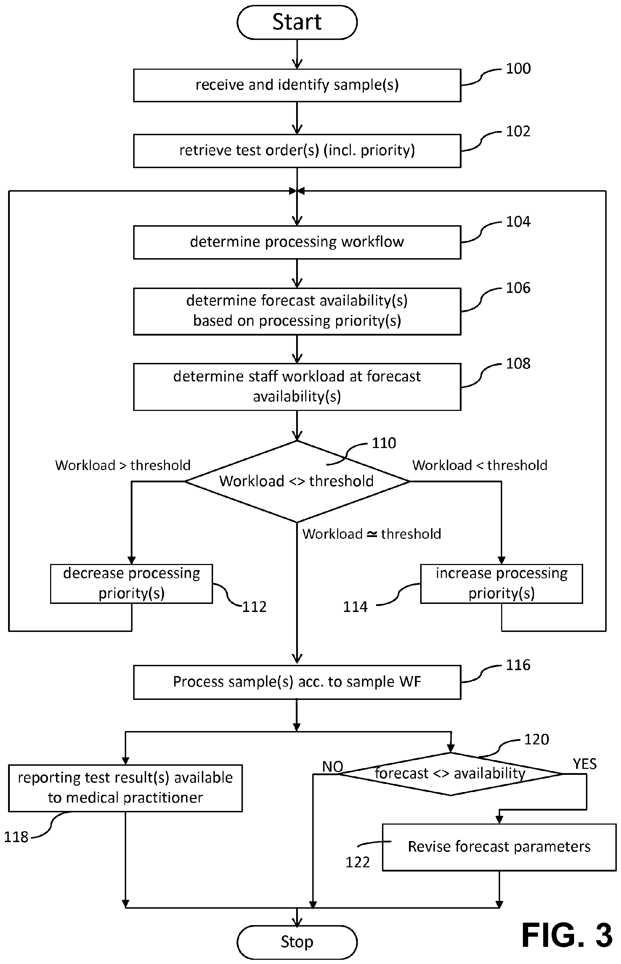

Figure 3 shows a flowchart illustrating an embodiment of the disclosed method for operating an analytical laboratory 1, wherein machine learning is employed to revise parameters for determining forecast availability of analytical results if the actual availability of analytical result(s) deviate from the forecast availability.

According to such further embodiments of the disclosed method/system, forecasting of the availability of analytical results corresponding to test orders is based on historical data and a plurality of weighted parameters. Accordingly, the method further comprising the steps:
- Recording actual availability of analytical result(s)
   In order to be able to evaluate the accuracy of the algorithm forecasting availability of analytical results, the actual time such are available is recorded as a bases of comparison.
- Recording data influencing the actual availability of analytical result(s) There are a number of factors that may influence the availability of the analytical results, comprising:
   - Actual processing time of the biological samples by at least one of the plurality of the laboratory instruments 10;
   - Actual validation time of a measurement
      Actual period of time required for the biological sample(s) to be transported from a point of collection from the patient to the analytical laboratory 1.
- If the actual availability of analytical result(s) deviates from the forecast availability of the same analytical results **(step 120),** then a correlation between the deviation of the actual availability of analytical result(s) from the forecast availability of the same analytical results and the influencing data is determined.
   For example if an analytical result actually becomes available 30 minutes later than forecast and at the same time the actual validation of the measurement took 30 minutes longer than predicted, then a correlation is determined.
- If a correlation is found, revising **(step 122)** one or more of the plurality of parameters used in the step of determining the forecast availability of analytical results.

Continuing on the example of the preceding bullet point, since a correlation is found between delayed availability of the analytical results and the validation time, the parameter used in forecasting the validation time (e.g. average/ mean) is adjusted in view of the experienced delay. According to various embodiments, extreme values are disregarded from consideration when revising parameters in order to avoid a single isolated occurrence from disrupting future calculations.

Actual in the context of the present application refers to real recorded values as opposed to predicted, forecast values. As such, actual values are recorded and not calculated.

According to further embodiments, in addition to determining a correlation between the deviation of the actual availability of analytical result(s) from the forecast availability of the same analytical results and the influencing data, a determination of causality/ root-cause analysis is also performed. Continuing further on the example of the paragraph above, if it is found that the laboratory instruments 10 finished processing the biological sample at e.g. 16:59 and knowing that laboratory personnel changes work shifts at 17:00, the cause for the delayed validation is determined to lie with the timing of the validation with respect to work shifts. As a remedy, parameters of the forecasting algorithm are adjusted to account for delays if a measurement needs to be validated in a time coinciding with a change of work shifts of laboratory personnel.

According to further embodiments, machine learning is extended to optimizing the determination of the staff workload respectively the workload threshold by analyzing the activities of the medical practitioners. As part thereof, a time the medical practitioner takes to consult a patient is recorded. Once a sufficient amount of records are available, a correlation is determined between a deviation of the actual time the medical practitioner takes to consult a patient versus the forecast time that was considered in determining the staff workload. Based on this correlation, parameters used in determining the staff workload respectively the workload threshold are adjusted iteratively to minimize the deviation. According to further embodiments, the prediction of the period of time from the forecast availability of analytical result(s) until a medical practitioner is to review the analytical result(s) is specific to a medical practitioner and is based on recorded work habits, such as average/ mean time to treat a patient. Additionally, the criticality of the patients is considered (based on the analytical results) to distinguish between an average/ mean time to treat a critical patient from the time to treat a mildly ill patient.

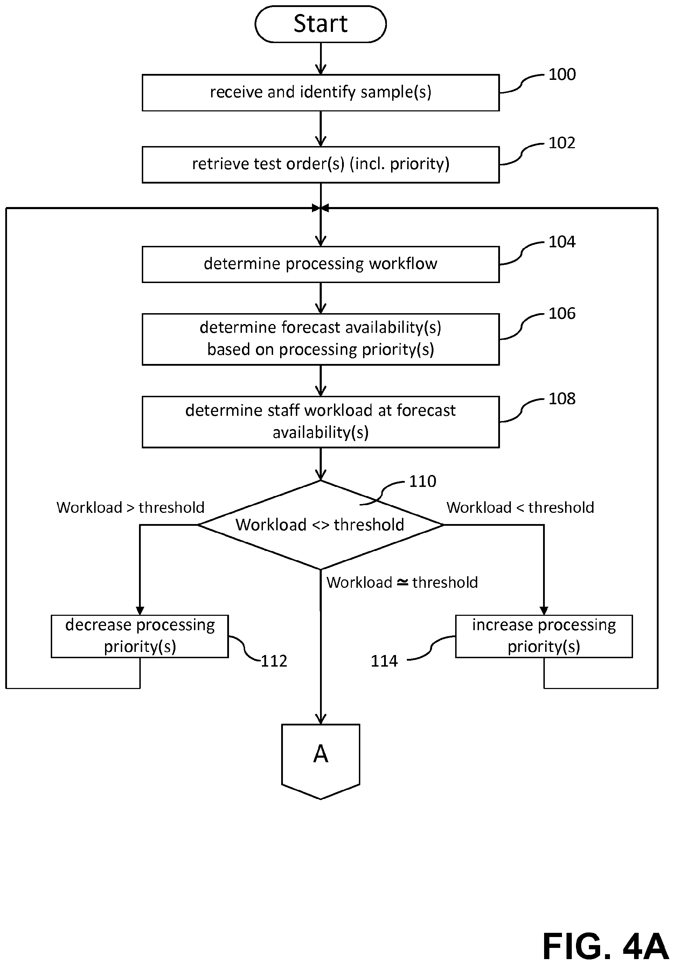

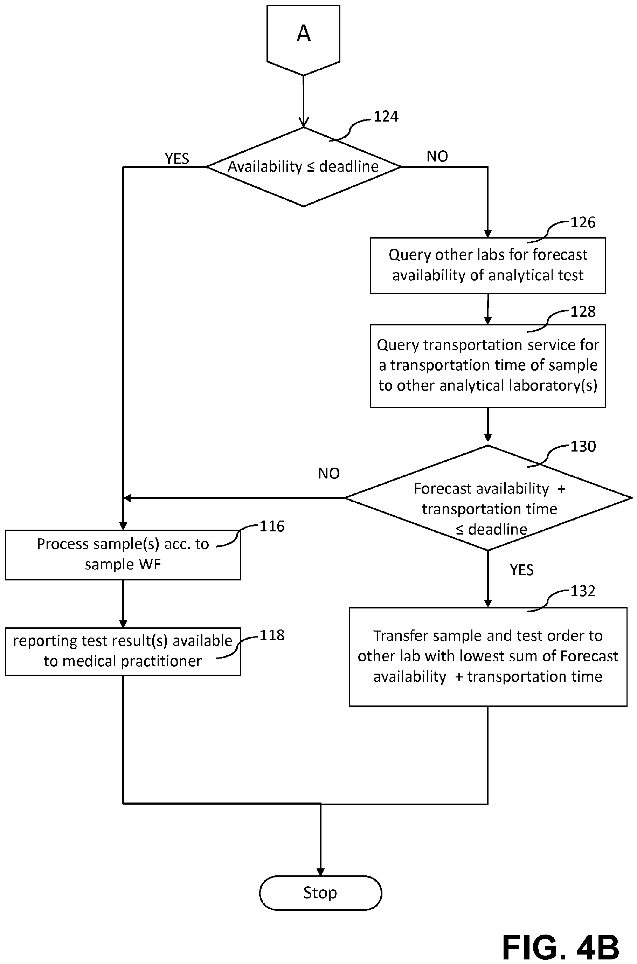

Figures 4A and 4B show a flowchart illustrating an embodiment of the disclosed method for operating an analytical laboratory, wherein the analytical laboratory 1 is in communicative connection with one or more other analytical laboratory(s). As illustrated on the flowchart, if the forecast availability of analytical result(s) corresponding to the test order(s) for a biological sample is beyond a specified availability deadline (specified in particular in the test order by the medical practitioner who issued the test order), then other analytical laboratories are contacted to evaluate if the test order can be completed in time by these. Hence, in a **step 124,** the control unit 20 determines whether the analytical laboratory 1 that received the sample is not able to complete a test order by a set deadline. If so, in a **step 126,** the control unit 20 sends a query to the other analytical laboratory(s) for providing a forecast of the availability of the analytical results corresponding to the test order(s) if the biological sample would be processed by the further analytical laboratory(s). Then in a **step 128,** a query is sent to one or more transportations services for a predicted transportation time of a sample to other analytical laboratory(s). Thereafter, in **step 130,** the control unit 20 determines the sum of the forecast availability and predicted transportation time for each of the other analytical laboratory(s) and in **step 132** transfers the biological sample to the other analytical laboratory for which the sum of the forecast availability and predicted transportation time are the lowest if lower than the specified availability deadline.

According to various embodiments, the biological sample is transferred to a further analytical laboratory by a transportation service either manually (e.g. courier or taxi) and/or automatically by means of a sample transportation system(e.g. a pneumatic tube or aerial transport system, such as a drone).

According to various embodiments, biological samples to be transferred to a particular further analytical laboratory are automatically transported by a sample transport system - such a conveyor- belt system - to a pick up station. According to an even further embodiment, the biological samples to be transferred to a particular further analytical laboratory are also packaged automatically - grouped by destination laboratory, in particular onto a sample rack comprising an RFID tag, wherein the control unit 20 records the corresponding test orders and processing priority onto the RFID tag.

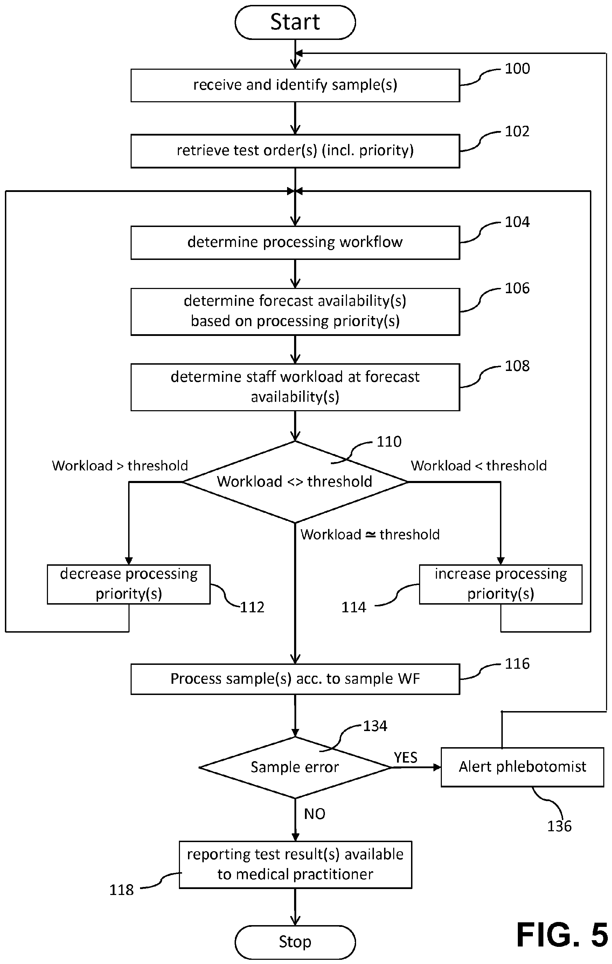

Figure 5 shows a flowchart of an embodiment of the disclosed method addressing situations when at least one analytical step of the test order(s) cannot be performed on a biological sample due to sample quality/ quantity. For saving time and resources, according to such embodiments, in a **step 134** the control unit 20 detects if an analytical step cannot be performed on a biological sample due to sample quality/ quantity. When this is the case, an alert is raised in a **step 136** indicating that a new sample needs to be provided for the analytical laboratory 1 to be able to carry out the test order(s). Thereafter the processing workflow for this sample will be recalculated once the new sample is received (step 100). According to further embodiments, the processing priority is increased for a test order as soon as a sample error is detected - in view of delays caused by the need to collect a new patient sample.

According to further embodiments, an alert is also sent (e.g. to a phlebotomist) if a test order is indicative of a repetitive analytical test (e.g. Troponin test for cardiac care) requiring repeated sample collection from a patient based on a schedule. To assist medical practitioners in collecting the patient samples in a timely manner, the alert is sent based on the schedule of the repetitive analytical test.

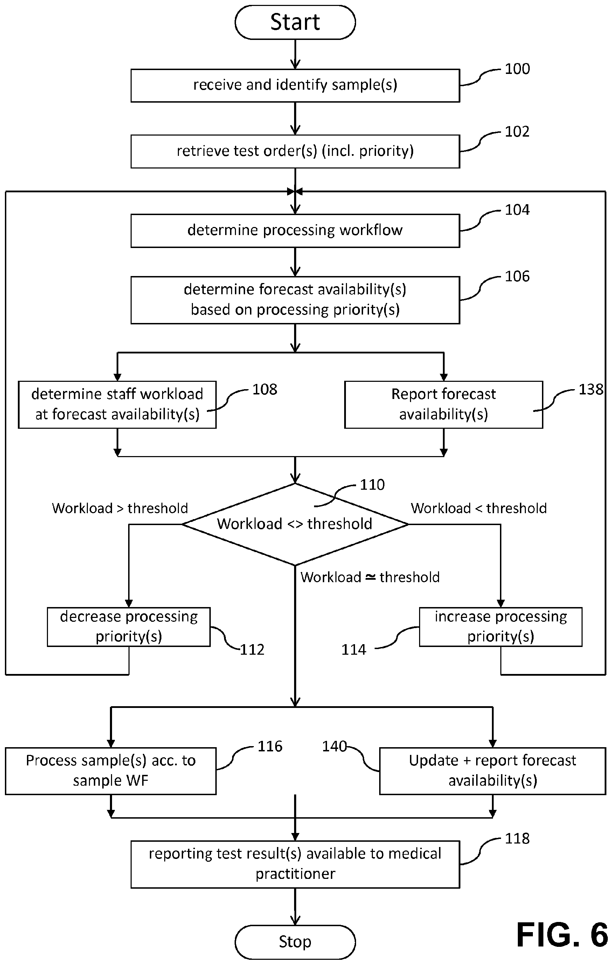

Figure 6 shows a flowchart illustrating an embodiment of the disclosed method for operating an analytical laboratory 1, wherein the forecast availability(s) of analytical result(s) is reported. In order to facilitate the planning activities of medical practitioners, as soon as the availability of an analytical result is determined, the forecast is reported **(step 138).** To ensure the medical practitioner has the most up-to-date information, in a **step 140** the forecast availability is updated during the processing of the biological sample and the updated forecast is reported.

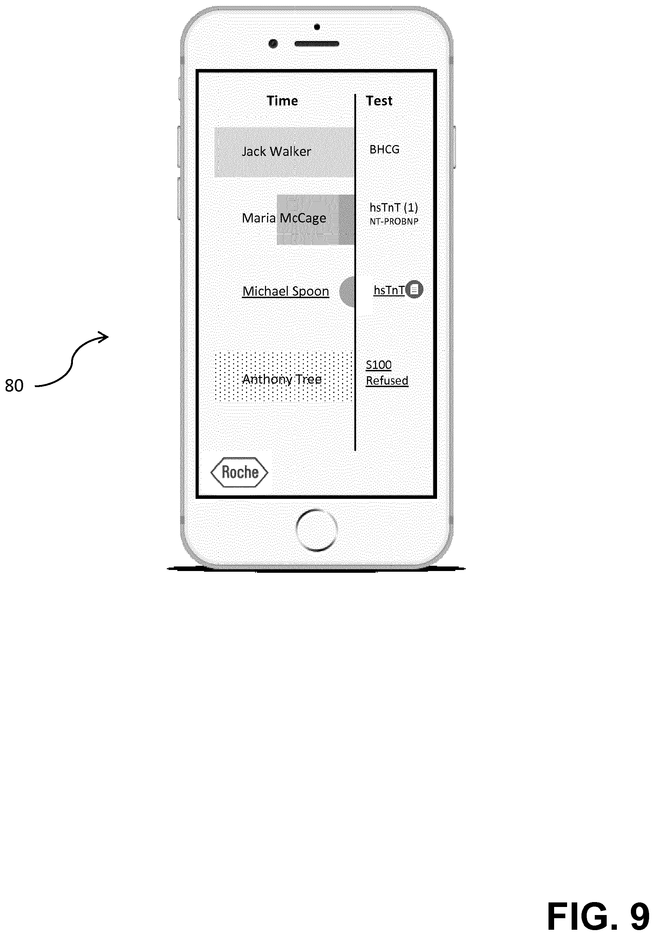

According to various embodiments of the disclosed method/ system reporting of forecast availability of analytical result(s) is via a user interface of a Hospital Information System 50, by a data transfer to a cloud server accessible by a mobile device application of a medical practitioner, by way of a printout or other suitable means. Figure 9 shows a mockup of an example of a mobile device application reporting the forecast availability of analytical results in the form of progress bars, which are updated (continuously or at certain intervals).

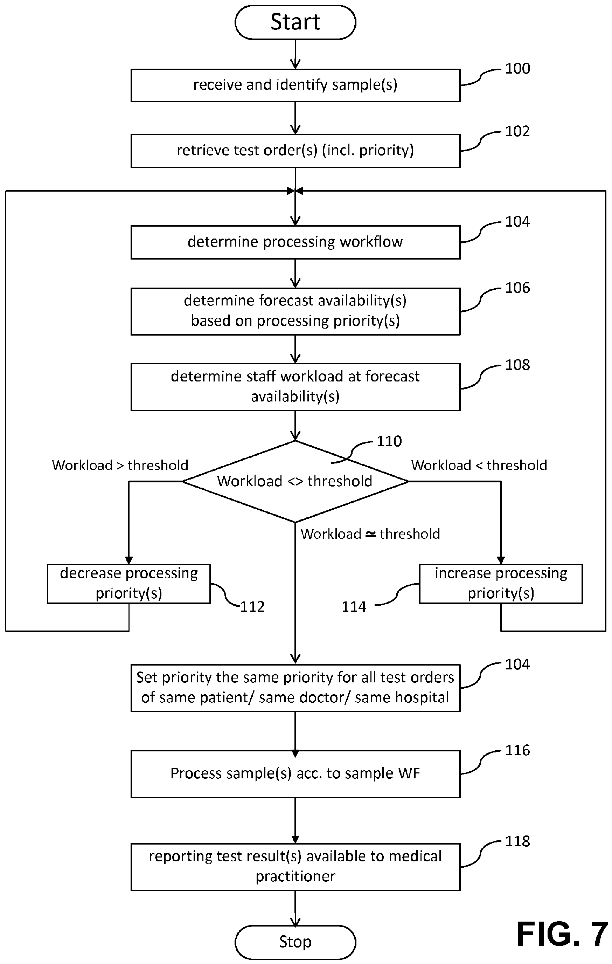

Figure 7 depicts a flowchart illustrating an embodiment of the disclosed method for operating an analytical laboratory 1, wherein the processing priority level of all test orders in a group is set to the highest level amongst the test orders of the group. Such grouping of test orders is based on one or more from the list comprising:
- Same patient
   In order to support medical practitioners making a diagnosis and treating a patient in view of the "full picture" - that is in view of all analytical results related to that patient, all test orders related to the same patient are prioritized equally, namely on the highest priority level amongst the test orders for the patient.
- Same medical practitioner who is to review the analytical results
   There are certain situations when there is a need for all test orders for a particular medical practitioner who is to review a number of analytical results to be available in the same time frame. The reasons include: medical practitioners who are known to always handle emergency cases, medical practitioners known to be present to review analytical results only for a limited amount of time.
- Same institution
   In order to support scientific studies, where a certain number of analytical test results are evaluated, embodiments of the disclosed method comprise the step of assigning the same processing priority for all test orders of the institution to ensure that all analytical results for the study are available in the same time frame and not one-by-one.

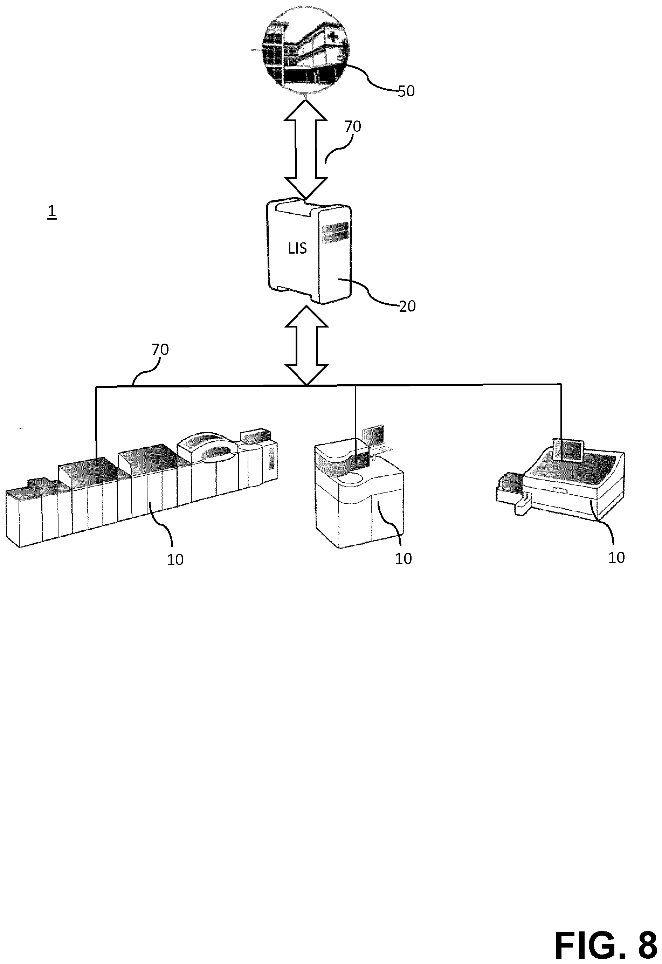

Turning now onto the block diagram of figure 8, embodiments of the disclosed analytical laboratory 1 for processing biological sample(s) comprise a plurality of laboratory instruments 10 and a control unit 20 communicatively connected by a communication network 70. The plurality of laboratory instruments 10 are configured to execute processing steps on the biological samples according to instructions from the control unit 20. According to embodiments of the disclosed analytical laboratory 1, the plurality of laboratory instruments 10 may be one or more instruments from the list comprising pre-analytical instruments, laboratory instrument(s) for loading samples comprising a sample input station, post-analytical instruments and also analytical instruments.

According to various embodiments of the disclosed analytical laboratory 1, the plurality of laboratory instruments 10 may be identical or different instruments such as clinical- & immunochemistry analyzers, coagulation chemistry analyzers, immunochemistry analyzers, urine analyzers, nucleic acid analyzers, hematology instruments etc.

According to further embodiments disclosed herein, in view of data privacy regulations, patient and/or medical practitioner's personal information is anonymized if required, ensuring that no patient and/or medical practitioner identity can be traced back.

Further disclosed and proposed is a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps as indicated above may be performed by using a computer or a computer network, preferably by using a computer program.

Further disclosed and proposed is a computer program product having program code means, in order to perform the method disclosed herein in one or more of the embodiments enclosed herein when the program is executed on a computer or computer network. Specifically, the program code means may be stored on a computer-readable data carrier.

Further disclosed and proposed is a data carrier having a data structure stored thereon, which, after loading into a computer or computer network, such as into a working memory or main memory of the computer or computer network, may execute the method according to one or more of the embodiments disclosed herein.

Further disclosed and proposed is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Further disclosed and proposed is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Furthermore, hereby disclosed and proposed are:
A computer or computer network comprising at least one processor, wherein the processor is adapted to perform the method according to one of the embodiments described in this description,
a computer loadable data structure that is adapted to perform the method according to one of the embodiments described in this description while the data structure is being executed on a computer,
a computer program, wherein the computer program is adapted to perform the method according to one of the embodiments described in this description while the program is being executed on a computer,
a computer program comprising program means for performing the method according to one of the embodiments described in this description while the computer program is being executed on a computer or on a computer network,
a computer program comprising program means according to the preceding embodiment, wherein the program means are stored on a storage medium readable to a computer,
a storage medium, wherein a data structure is stored on the storage medium and wherein the data structure is adapted to perform the method according to one of the embodiments described in this description after having been loaded into a main and/or working storage of a computer or of a computer network, and
a computer program product having program code means, wherein the program code means can be stored or are stored on a storage medium, for performing the method according to one of the embodiments described in this description, if the program code means are executed on a computer or on a computer network.

It will be understood that many variations could be adopted based on the specific structure hereinbefore described without departing from the scope of the invention as defined in the following claims.

### REFERENCE LIST:

| | |
|---|---|
| analytical laboratory | 1 |
| laboratory instrument | 10 |
| control unit | 20 |
| Hospital Information System | 50 |
| communication network | 70 |
| mobile device application for reporting forecast availability | 80 |
| receive and identify sample(s) | step 100 |
| retrieve test order(s) (incl. priority) | step 102 |
| setting processing priority based on objective urgency level | step 103 |
| determine processing workflow | step 104 |
| determining a forecast availability of analytical result(s) | step 106 |
| determine staff workload at forecast availability(s) | step 108 |
| comparison of staff workload and workload threshold | step 110 |
| decreasing processing priority | step 112 |
| increasing processing priority | step 114 |
| process sample(s) acc. to sample WF | step 116 |
| reporting test result(s) available to medical practitioner | step 118 |
| determining deviation between forecast and actual availability | step 120 |
| Revise forecast parameters | step 122 |
| determining if forecast availability before deadline | step 124 |
| query other labs for forecast availability of analytical test | ste p 126 |
| query transportation service for a transportation time of sample to other analytical laboratory(s) | step 128 |
| determining if sum of forecast availability and transportation time is less than deadline | step 130 |
| transfer sample to different lab | step 132 |
| determine sample error | step 134 |
| alert phlebotomist | step 136 |
| report forecast availability | step 138 |
| Update + report forecast availability(s) | step 140 |

## Claims

1. A computer implemented method for operating an analytical laboratory (1), comprising a plurality of laboratory instruments (10) configured to obtain a measurement value indicative of a characteristic of the biological sample, the method comprising the steps of:
- receiving and identifying a plurality of biological samples;
- retrieving one or more test orders corresponding to each biological sample, each test order defining at least one analytical step to be carried out on the biological sample by at least one of the plurality of the laboratory instruments (10), each test order comprising a processing priority;
- determining a processing workflow for processing each biological sample based on corresponding test order(s) and corresponding processing priority, wherein the processing workflow is determined such that test order(s) with higher processing priority are completed before test order(s) with lower processing priority;
- determining a forecast availability of analytical result(s) corresponding to each test order;
**characterized in that** the method comprises the steps of:
- determining a staff workload and a workload threshold at the time of forecast availability corresponding to each test order;
- decreasing the processing priority corresponding to the respective test order if the staff workload is greater than a workload threshold;
- increasing the processing priority corresponding to the respective test order if the staff workload is lower than the workload threshold;
- controlling the plurality of laboratory instruments (10) of the analytical laboratory (1) to process each biological sample according to the corresponding processing workflow and reporting test results obtained by the at least one analytical step on the biological samples performed by the at least one of the plurality of the laboratory instruments (10) if the staff workload matches the workload threshold.

2. The method according to claim 1, wherein the staff workload and workload threshold are indicative of one or more from the list comprising:
- a number of results a medical practitioner is to review before reviewing the test result corresponding to the respective test order, and/or
- a predicted period of time from the forecast availability of analytical result(s) until a medical practitioner is to review the analytical result(s);
the staff workload corresponding to a test order for the biological sample of a patient in critical condition being always 0, wherein a staff workload of 0 ensures that the processing priority of corresponding test orders are increased to a highest level of processing priority.

3. The method according to claim 1 or 2, further comprising the step of:
setting the priority of a test order in view of an urgency level determined based on one or more from the list comprising:
- processing priority level of the test order as retrieved;
- an indication of patient criticality;
- analytical test class;
- patient condition progress/change, in particular based on patient monitoring;
- source of test order;
- a status of one or more of the plurality of laboratory instruments (10).

4. The method according to one of the claims 1 to 3, further comprising the step of re-determining a processing workflow for processing each biological sample and re-determining the forecast availability of an analytical result corresponding to each test order each time the processing priority of one or more test order(s) is increased or decreased.

5. The method according to one of the claims 1 to 4, wherein determining a forecast availability of an analytical result comprises one or more steps from the list comprising:
- determining a predicted processing time of the biological samples by the laboratory instrument(s) (10);
- determining a predicted validation time, wherein the validation time is determined in view of a schedule of a laboratory staff responsible for validation of analytical test results;
- determining a predicted period of time required for the biological sample(s) to be transported from a point of collection from the patient to the analytical laboratory (1);
- determining delays due interdependency(s) between analytical step(s) and analytical results.

6. The method according to one of the claims 1 to 5, further comprising the step of reporting the forecast availability(s) of analytical result(s) upon being determined and/or re-determined.

7. The method according to one of the claims 1 to 6, further comprising the step of setting the processing priority level of all test orders to the highest level amongst the test orders corresponding to:
- same patient;
- same medical practitioner who is to review the analytical results corresponding to each test order;
- same institution as originator of the test orders.

8. The method according to one of the claims 1 to 7, wherein forecasting of the availability of an analytical result corresponding to each test order is based on historical data and a plurality of weighted parameters, the method further comprising the steps:
- recording actual availability of analytical result(s);
- recording data influencing the actual availability of analytical result(s), said data comprising:
- actual processing time of the biological samples by at least one of the plurality of the laboratory instruments (10);
- actual validation time;
- actual period of time required for the biological sample(s) to be transported from a point of collection from the patient to the analytical laboratory (1);
- determining a correlation between a deviation of the actual availability of analytical result(s) from the forecast availability of the same analytical results and the influencing data and revising one or more of the plurality of parameters used in the step of determining the forecast availability of analytical results if the actual availability of analytical result(s) deviate from the forecast availability of the same analytical results based on said correlation.

9. The method according to claim 8 further comprising the steps of:
- recording a time the medical practitioner takes to consult a patient;
- determining a deviation of the actual time the medical practitioner takes to consult a patient versus the forecast time that was considered in determining the staff workload.

10. The method according to one of the claims 1 to 9, wherein if the forecast availability of analytical result(s) corresponding to the test order(s) for a biological sample is beyond a specified availability deadline, the method further comprises the steps of:
- query one or more other analytical laboratory(s) for a forecast availability by the further analytical laboratory(s) of analytical results corresponding to the test order(s);
- query a transportation service for a predicted transportation time of the biological sample to said other analytical laboratory(s);
- transferring the biological sample(s) to the other analytical laboratory for which the sum of the forecast availability and predicted transportation time is the lowest if lower than the specified availability deadline.

11. The method according to one of the claims 1 to 10, further comprising the step of generating an alert if at least one analytical step of the test order(s) cannot be performed on a biological sample due to its quality and/or quantity, said alert being indicative that a new sample need to be provided for the analytical laboratory (1) to be able to carry out the test order(s) related to the biological sample.

12. An analytical laboratory (1), the analytical laboratory (1) comprising:
- a plurality of laboratory instruments (10) for processing biological samples; and
- a control unit (20),
the plurality of laboratory instruments (10) and the control unit (20) being communicatively connected by a communication network (70), wherein the control unit (20) is communicatively connected by a communication network (70) to a Hospital Information System (50) and wherein the control unit is configured to carry out the method according to one of the claims 1 through 11.

13. A computer program product comprising instructions which, when executed by a computer system, control an analytical laboratory to perform the steps of any one of the methods according to claims 1 through 11.

## Patentansprüche

1. Computerimplementiertes Verfahren zum Betreiben eines analytischen Labors (1), umfassend eine Vielzahl von Laborinstrumenten (10), die dafür ausgebildet sind, einen Messwert zu erhalten, der ein Merkmal der biologischen Probe angibt, wobei das Verfahren die folgenden Schritte umfasst:
- Aufnehmen und Identifizieren einer Vielzahl von biologischen Proben;
- Abrufen eines oder mehrerer Testaufträge, die jeder biologischen Probe entsprechen, wobei jeder Testauftrag mindestens einen analytischen Schritt definiert, der von mindestens einem der Vielzahl der Laborinstrumente (10) an der biologischen Probe ausgeführt werden soll, wobei jeder Testauftrag eine Bearbeitungspriorität umfasst;
- Bestimmen eines Bearbeitungs-Workflows zum Bearbeiten jeder biologischen Probe basierend auf einem entsprechenden Testauftrag/Testaufträgen und entsprechender Bearbeitungspriorität, wobei der Bearbeitungs-Workflow so bestimmt wird, dass ein Testauftrag/Testaufträge mit höherer Bearbeitungspriorität vor einem Testauftrag/Testaufträgen mit niedrigerer Bearbeitungspriorität abgeschlossen wird/werden;
- Bestimmen einer voraussichtlichen Verfügbarkeit von einem analytischen Ergebnis/Ergebnissen, die jedem Testauftrag entspricht;
**dadurch gekennzeichnet, dass** das Verfahren die folgenden Schritte umfasst:
- Bestimmen einer Arbeitsbelastung des Personals und einer Arbeitsbelastungsschwelle zum Zeitpunkt der voraussichtlichen Verfügbarkeit, die jedem Testauftrag entspricht;
- Senken der Bearbeitungspriorität, die dem jeweiligen Testauftrag entspricht, wenn die Arbeitsbelastung des Personals höher ist als eine Arbeitsbelastungsschwelle;
- Anheben der Bearbeitungspriorität, die dem jeweiligen Testauftrag entspricht, wenn die Arbeitsbelastung des Personals geringer ist als die Arbeitsbelastung s schwelle;
- Steuern der Vielzahl von Laborinstrumenten (10) des analytischen Labors (1) derart, dass sie jede biologische Probe gemäß dem entsprechenden Bearbeitungs-Workflow bearbeiten, und Berichten von Testergebnissen, die mit dem mindestens einen analytischen Schritt an den biologischen Proben erhalten wurden, der von dem mindestens einen der Vielzahl der Laborinstrumente (10) ausgeführt wurde, wenn die Arbeitsbelastung des Personals mit der Arbeitsbelastungsschwelle übereinstimmt.

2. Verfahren nach Anspruch 1, wobei die Arbeitsbelastung des Personals und die Arbeitsbelastungsschwelle eines oder mehrere aus der Liste angibt, die Folgendes umfasst:
- eine Anzahl von Ergebnissen, die ein praktischer Arzt überprüfen muss, bevor er das Testergebnis überprüft, das dem jeweiligen Testauftrag entspricht, und/oder
- einen vorhergesagten Zeitraum ausgehend von der voraussichtlichen Verfügbarkeit eines analytischen Ergebnisses/analytischer Ergebnisse, bis ein praktischer Arzt das/die analytische(n) Ergebnis/Ergebnisse überprüfen muss;
wobei die Arbeitsbelastung des Personals, die einem Testauftrag für die biologische Probe eines Patienten in kritischem Zustand entspricht, immer 0 ist, wobei eine Arbeitsbelastung des Personals von 0 sicherstellt, dass die Bearbeitungspriorität entsprechender Testaufträge auf eine höchste Bearbeitungsprioritätsstufe angehoben wird.

3. Verfahren nach Anspruch 1 oder 2, ferner umfassend den folgenden Schritt: Festlegen der Priorität eines Testauftrages im Hinblick auf eine Dringlichkeitsstufe, die basierend auf einem oder mehreren aus der Liste bestimmt wird, die Folgendes umfasst:
- Bearbeitungsprioritätsstufe des überprüften Testauftrags;
- eine Angabe zur Patientenkritikalität;
- analytische Testklasse;
- Verlauf/Veränderung des Patientenzustands, insbesondere basierend auf Patientenüberwachung;
- Quelle des Testauftrags;
- einen Status von einem oder mehreren der Vielzahl von Laborinstrumenten (10).

4. Verfahren nach einem der Ansprüche 1 bis 3, ferner umfassend den Schritt des erneuten Bestimmens eines Bearbeitungs-Workflows zum Bearbeiten jeder biologischen Probe und erneuten Bestimmens der voraussichtlichen Verfügbarkeit eines analytischen Ergebnisses, die jedem Testauftrag entspricht, immer dann, wenn die Bearbeitungspriorität eines Testauftrags oder mehrerer Testaufträge angehoben oder gesenkt wird.

5. Verfahren nach einem der Ansprüche 1 bis 4, wobei das Bestimmen einer voraussichtlichen Verfügbarkeit eines analytischen Ergebnisses einen oder mehrere Schritt(e) aus der Liste umfasst, die Folgendes umfasst:
- Bestimmen einer geschätzten Bearbeitungszeit für die biologischen Proben durch das/die Laborinstrument(e) (10);
- Bestimmen einer geschätzten Bewertungszeit, wobei die Bewertungszeit hinsichtlich eines Arbeitsplans eines Laborpersonals bestimmt wird, das für die Bewertung analytischer Testergebnisse verantwortlich ist;
- Bestimmen eines geschätzten Zeitraums, der zum Transportieren der biologischen Probe(n) von einem Entnahmepunkt von dem Patienten zu dem analytischen Labor (1) erforderlich ist;
- Bestimmen von Verzögerungen aufgrund wechselseitiger Abhängigkeit(en) zwischen einem analytischen Schritt(en) und analytischen Ergebnissen.

6. Verfahren nach einem der Ansprüche 1 bis 5, ferner umfassend den Schritt des Berichtens der voraussichtlichen Verfügbarkeit(en) eines analytischen Ergebnisses/analytischer Ergebnisse, nachdem es/sie bestimmt und/oder erneut bestimmt worden ist/sind.

7. Verfahren nach einem der Ansprüche 1 bis 6, ferner umfassend den Schritt des Festlegens der Bearbeitungsprioritätsstufe aller Testaufträge auf die höchste Stufe unter den Testaufträgen, die Folgendem entspricht:
- demselben Patienten;
- demselben praktischen Arzt, der die jedem Testauftrag entsprechenden analytischen Ergebnisse überprüfen muss;
- derselben Institution wie der Urheber der Testaufträge.

8. Verfahren nach einem der Ansprüche 1 bis 7, wobei die Vorhersage der Verfügbarkeit eines analytischen Ergebnisses, die jedem Testauftrag entspricht, auf historischen Daten und einer Vielzahl von gewichteten Parametern basiert, wobei das Verfahren ferner die folgenden Schritte umfasst:
- Aufzeichnen der tatsächlichen Verfügbarkeit eines analytischen Ergebnisses/analytischer Ergebnisse;
- Aufzeichnen von Daten, die die tatsächliche Verfügbarkeit eines analytischen Ergebnisses/analytischer Ergebnisse beeinflussen, wobei die Daten Folgendes umfassen:
- tatsächliche Bearbeitungszeit für die biologischen Proben durch mindestens eines der Vielzahl von Laborinstrumenten (10);
- tatsächliche Bewertungszeit;
- tatsächlicher Zeitraum, der zum Transportieren der biologischen Probe(n) von einem Entnahmepunkt von dem Patienten zu dem analytischen Labor (1) erforderlich ist;
- Bestimmen einer Korrelation zwischen einer Abweichung der tatsächlichen Verfügbarkeit eines analytischen Ergebnisses/analytischer Ergebnisse von der voraussichtlichen Verfügbarkeit derselben analytischen Ergebnisse und den beeinflussenden Daten und Überarbeiten eines oder mehrerer der Vielzahl von Parametern, die in dem Schritt des Bestimmens der voraussichtlichen Verfügbarkeit analytischer Ergebnisse genutzt werden, wenn die tatsächliche Verfügbarkeit eines analytischen Ergebnisses/analytischer Ergebnisse von der voraussichtlichen Verfügbarkeit derselben analytischen Ergebnisse basierend auf der Korrelation abweicht.

9. Verfahren nach Anspruch 8, ferner umfassend die folgenden Schritte:
- Aufzeichnen einer Zeit, die der praktische Arzt zur Konsultation eines Patienten benötigt;
- Bestimmen einer Abweichung der tatsächlichen Zeit, die der praktische Arzt zur Konsultation eines Patienten benötigt, gegenüber der geschätzten Zeit, die beim Bestimmen der Arbeitsbelastung des Personals berücksichtigt wurde.

10. Verfahren nach einem der Ansprüche 1 bis 9, wobei, wenn die voraussichtliche Verfügbarkeit eines analytischen Ergebnisses/analytischer Ergebnisse, die dem/den Testauftrag/Testaufträgen für eine biologische Probe entspricht, über eine spezifizierte Verfügbarkeitsfrist hinausgeht, das Verfahren ferner die folgenden Schritte umfasst:
- Anfragen bei einem oder mehreren anderen analytischen Labor(en) nach einer voraussichtlichen Verfügbarkeit von dem/den weiteren analytischen Labor(en) von analytischen Ergebnissen, die dem/den Testauftrag/Testaufträgen entspricht;
- Anfragen bei einem Transportdienstleister nach einer geschätzten Transportzeit der biologischen Probe an das/die andere(n) analytische(n) Labor(e);
- Überführen der biologischen Probe(n) zu dem anderen analytischen Labor, für das die Summe der voraussichtlichen Verfügbarkeit und geschätzten Transportzeit die niedrigste ist, wenn sie geringer ist als die spezifizierte Verfügbarkeitsfrist.

11. Verfahren nach einem der Ansprüche 1 bis 10, ferner umfassend den Schritt des Erzeugens eines Alarms, wenn mindestens ein analytischer Schritt des/der Testauftrags/Testaufträge aufgrund ihrer Qualität und/oder Quantität nicht an einer biologischen Probe ausgeführt werden kann, wobei der Alarm angibt, dass eine neue Probe für das analytische Labor (1) bereitgestellt werden muss, damit der/die Testauftrag/Testaufträge bezüglich der biologischen Probe ausgeführt werden kann/können.

12. Analytisches Labor (1), wobei das analytische Labor (1) Folgendes umfasst:
- eine Vielzahl von Laborinstrumenten (10) zum Bearbeiten biologischer Proben; und
- eine Steuereinheit (20),
wobei die Vielzahl von Laborinstrumenten (10) und die Steuereinheit (20) durch ein Kommunikationsnetzwerk (70) kommunikativ verbunden sind, wobei die Steuereinheit (20) durch ein Kommunikationsnetzwerk (70) kommunikativ mit einem Krankenhaus-Informationssystem (50) verbunden ist und wobei die Steuereinheit dafür ausgebildet ist, das Verfahren nach einem der Ansprüche 1 bis 11 auszuführen.

13. Computerprogrammprodukt, das Anweisungen umfasst, die bei Ausführung von einem Computersystem ein analytisches Labor derart steuern, dass es die Schritte eines der Verfahren nach den Ansprüchen 1 bis 11 ausführt.

## Revendications

1. Procédé mis en œuvre par ordinateur destiné au fonctionnement d'un laboratoire d'analyse (1), comprenant une pluralité d'instruments de laboratoire (10) conçus pour obtenir une valeur de mesure indiquant une caractéristique de l'échantillon biologique, le procédé comprenant les étapes suivantes :
- la réception et l'identification d'une pluralité d'échantillons biologiques ;
- la récupération d'au moins une commande de test correspondant à chaque échantillon biologique, chaque commande de test définissant au moins une étape d'analyse à effectuer sur l'échantillon biologique par au moins un instrument de la pluralité d'instruments de laboratoire (10), chaque commande de test comprenant une priorité de traitement ;
- la détermination d'un flux de traitement pour traiter chaque échantillon biologique en fonction de l'au moins une commande de test correspondante et de la priorité de traitement correspondante, dans lequel le flux de traitement est déterminé de telle sorte que l'au moins une commande de test présentant une priorité élevée de traitement est terminée avant l'au moins une commande de test présentant une priorité faible de traitement ;
- la détermination d'une disponibilité prévue d'au moins un résultat d'analyse correspondant à chaque commande de test ;
**caractérisé en ce que** le procédé comprend les étapes suivantes :
- la détermination d'une charge de travail du personnel et d'un seuil de charge de travail au moment de la disponibilité prévue correspondant à chaque commande de test ;
- la réduction de la priorité de traitement correspondant à la commande de test respective lorsque la charge de travail du personnel est supérieure à un seuil de charge de travail ;
- l'augmentation de la priorité de traitement correspondant à la commande de test respective lorsque la charge de travail du personnel est inférieure au seuil de charge de travail ;
- la commande de la pluralité d'instruments de laboratoire (10) du laboratoire d'analyse (1) pour traiter chaque échantillon biologique selon le flux de traitement correspondant et pour rapporter les résultats de test obtenus selon l'au moins une étape d'analyse sur les échantillons biologiques effectuée par l'au moins un instrument de la pluralité d'instruments de laboratoire (10) lorsque la charge de travail du personnel correspond au seuil de charge de travail.

2. Procédé selon la revendication 1, dans lequel la charge de travail du personnel et le seuil de charge de travail indiquent des éléments de la liste comprenant :
- un certain nombre de résultats devant être vérifiés par un médecin praticien avant la vérification du résultat de test correspondant à la commande de test respective, et/ou
- une période de temps prédite à partir de la disponibilité prévue de l'au moins un résultat d'analyse jusqu'à ce qu'un médecin praticien vérifie l'au moins un résultat d'analyse ;
la charge de travail correspondant à une commande de test pour l'échantillon biologique d'un patient dans un état critique étant toujours 0, dans lequel une charge de travail de personnel de 0 assure que la priorité de traitement des commandes de test correspondantes est augmentée à un niveau le plus élevé de priorité de traitement.

3. Procédé selon la revendication 1 ou 2, comprenant en outre l'étape suivante : la définition de la priorité d'une commande de test compte tenu d'un niveau d'urgence déterminé en fonction des éléments de la liste comprenant :
- un niveau de priorité de traitement de la commande de test telle que récupérée ; et/ou
- une indication de gravité du patient ; et/ou
- une classe de test d'analyse ; et/ou
- une évolution / un changement de l'état du patient, en particulier en fonction de la surveillance du patient ; et/ou
- une source de la commande de test ; et/ou
- un état de l'au moins un instrument de la pluralité d'instruments de laboratoire (10).

4. Procédé selon l'une quelconque des revendications 1 à 3, comprenant en outre l'étape de redétermination d'un flux de traitement pour traiter chaque échantillon biologique et redéterminer la disponibilité prévue d'un résultat d'analyse correspondant à chaque commande de test à chaque fois que la priorité de traitement d'une ou de plusieurs commandes de test est augmentée ou réduite.

5. Procédé selon l'une quelconque des revendications 1 à 4, dans lequel la détermination d'une disponibilité prévue d'un résultat d'analyse comprend des étapes de la liste comprenant :
- la détermination d'un temps de traitement prédit des échantillons biologiques par l'au moins un instrument de laboratoire (10) ; et/ou
- la détermination d'un temps de validation prédit, dans lequel le temps de validation est déterminé compte tenu d'un planning d'un personnel de laboratoire responsable de la validation des résultats de test d'analyse ; et/ou
- la détermination d'une période de temps prédite nécessaire pour transporter l'au moins un échantillon biologique depuis un point de collecte chez le patient jusqu'au laboratoire d'analyse (1) ; et/ou
- la détermination des retards en raison d'au moins une interdépendance entre l'au moins une étape d'analyse et les résultats d'analyse.

6. Procédé selon l'une quelconque des revendications 1 à 5, comprenant en outre l'étape de rapport de l'au moins une disponibilité prévue de l'au moins un résultat d'analyse dès qu'elle a été déterminée et/ou redéterminée.

7. Procédé selon l'une quelconque des revendications 1 à 6, comprenant en outre l'étape de définition du niveau de priorité de traitement de toutes les commandes de test au niveau le plus élevé parmi les commandes de test correspondant :
- au même patient ;
- au même médecin praticien, lequel doit vérifier les résultats d'analyse correspondant à chaque commande de test ;
- à la même institution en tant qu'émetteur des commandes de test.

8. Procédé selon l'une quelconque des revendications 1 à 7, dans lequel la prévision de la disponibilité d'un résultat d'analyse correspondant à chaque commande de test se base sur des données d'historique et une pluralité de paramètres pondérés, le procédé comprenant en outre les étapes suivantes :
- l'enregistrement de la disponibilité réelle de l'au moins un résultat d'analyse ;
- l'enregistrement des données influençant la disponibilité réelle de l'au moins un résultat d'analyse, lesdites données comprenant :
- le temps de traitement réel des échantillons biologiques par l'au moins un instrument de la pluralité d'instruments de laboratoire (10) ;
- le temps de validation réel ;
- la période de temps réelle nécessaire pour transporter l'au moins un échantillon biologique depuis un point de collecte chez le patient jusqu'au laboratoire d'analyse (1) ;
- la détermination d'une corrélation entre un écart de la disponibilité réelle de l'au moins un résultat d'analyse avec la disponibilité prévue des mêmes résultats d'analyse et les données présentant une influence et la vérification de l'au moins un paramètre de la pluralité de paramètres utilisés dans l'étape de détermination de la disponibilité prévue des résultats d'analyse lorsque la disponibilité réelle de l'au moins un résultat d'analyse s'écarte de la disponibilité prévue des mêmes résultats d'analyse sur la base de ladite corrélation.

9. Procédé selon la revendication 8, comprenant en outre les étapes suivantes :
- l'enregistrement d'un temps nécessaire au médecin praticien pour s'entretenir avec un patient ;
- la détermination d'un écart entre le temps réel nécessaire au médecin praticien pour s'entretenir avec un patient et le temps prévu, lequel a été pris en compte pour déterminer la charge de travail du personnel.

10. Procédé selon l'une quelconque des revendications 1 à 9, dans lequel lorsque la disponibilité prévue de l'au moins un résultat d'analyse correspondant à l'au moins une commande de test pour un échantillon biologique se situe au-delà d'un délai de disponibilité spécifié, le procédé comprend en outre les étapes suivantes :
- l'interrogation d'au moins un autre laboratoire d'analyse concernant une disponibilité prévue par l'au moins un autre laboratoire d'analyse des résultats d'analyse correspondant à l'au moins une commande de test ;
- l'interrogation d'un service de transport concernant un temps de transport prédit de l'échantillon biologique jusqu'à l'au moins un autre laboratoire d'analyse ;
- le transfert de l'au moins un échantillon biologique vers l'autre laboratoire d'analyse pour lequel la somme de la disponibilité prévue et du temps de transport prédit est la plus faible lorsqu'elle est inférieure au délai de disponibilité spécifiée.

11. Procédé selon l'une quelconque des revendications 1 à 10, comprenant en outre l'étape de génération d'une alerte lorsque l'au moins une étape d'analyse de l'au moins une commande de test ne peut pas être réalisée sur un échantillon biologique en raison de sa qualité et/ou de sa quantité, ladite alerte indiquant qu'un nouvel échantillon doit être prélevé afin que le laboratoire d'analyse (1) puisse effectuer l'au moins une commande de test concernant l'échantillon biologique.

12. Laboratoire d'analyse (1), le laboratoire d'analyse (1) comprenant :
- une pluralité d'instruments de laboratoire (10) pour traiter des échantillons biologiques ; et
- une unité de commande (20),
la pluralité d'instruments de laboratoire (10) et l'unité de commande (20) étant connectés en communication par le biais d'un réseau de communication (70), dans lequel l'unité de commande (20) est connectée en communication par le biais d'un réseau de communication (70) avec un système d'information hospitalier (50) et dans lequel l'unité de commande est conçue pour exécuter le procédé selon l'une quelconque des revendications 1 à 11.

13. Produit de programme informatique comprenant des instructions, lesquelles, lorsqu'elles sont exécutées par un système informatique, commandent un laboratoire d'analyse pour mettre en œuvre les étapes de l'un quelconque des procédés selon les revendications 1 à 11.
